# EUROPEAN PATENT APPLICATION

(11) **EP 4 813 310 A1**
(43) Date of publication of application: **30.09.2026**
(21) Application number: 25165957.9
(22) Date of filing: 25.03.2025
(51) Int. Cl.: A61B 6/08, A61B 6/00, A61B 6/58

(54) **METHOD FOR SETTING A RADIOGRAPHIC IMAGING SYSTEM**

(71) Applicant: Koninklijke Philips N.V., 5656 AE Eindhoven (NL)
(72) Inventor: MENSER, Bernd, Eindhoven (NL); MAY, Jan Marek, Eindhoven (NL); FEIERABEND, Volker, 5656AG Eindhoven (NL); SCHLUETER, Mathias, Eindhoven (NL)
(74) Representative: Philips Intellectual Property & Standards

(57) **Abstract**

The subject-matter of the present disclosure relates to a computer implemented method for setting a radiographic imaging system, the method comprising: (i) arranging the radiographic imaging system in a first configuration; (ii) obtaining a reference area, based on a known geometry of the radiographic imaging system with respect to the reference area; (iii) determining a first collimation transformation based on an irradiated area in a first image and mapping the irradiated area onto the reference area; and (iv) determining a second collimation transformation by identifying an illuminated area in a second image by mapping the illuminated area onto the reference area.

## Description

### FIELD OF THE INVENTION

The subject-matter of the present disclosure relates to radiography, and in particular obtaining accurate images from a radiographic imaging system, and a radiographic imaging system therefor.

### BACKGROUND OF THE INVENTION

Radiographic imaging systems, such as diagnostic systems and interventional systems, include various components that can be moved relative to each other and their environment. To ensure the imaging of these systems is of a suitable quality, it is important to ensure that components are suitably aligned. The components of the systems can be arranged in various configurations, which are defined by system geometry parameters.

Radiographic imaging systems typically include a collimator to change the size of a beam of radiation emitted by a radiation generator. The collimator may be a motorised automatic collimator or a manual collimator. Collimators are used to limit radiation exposure to a target area, i.e., to reduce the amount of radiation to which a patient or an operator of the system is exposed, and to reduce the amount of scatter radiation. The collimator includes collimator shutters which are used to control the beam of radiation, e.g., to change the size of the beam of radiation. In the case of a motorised automatic collimator, the collimator shutters can be moved automatically into a position. The position and orientation of the collimator shutters can be measured and stored as system geometry parameters. The system geometry parameters can be used to predict an area of imaging.

However, due to mechanical tolerances, measurement uncertainties and limited precision of motor positioning, the predicted area of imaging and an area irradiated by the radiation beam, can differ significantly. This reduces the accuracy and consistency of predicting an area of imaging, leading to reduced image quality, increased patient dose or even re-takes due to cropped anatomy.

It is therefore an aim of the subject-matter of the present disclosure to improve on the prior art.

### SUMMARY OF THE INVENTION

According to a first aspect of the present invention, there is provided a computer implemented method for setting corrective transformations for a radiographic imaging system, wherein the radiographic imaging system comprises a first image sensor and a second image sensor, the method comprising: (i) arranging the radiographic imaging system in a first configuration; (ii) obtaining a reference area, based on a known geometry of the radiographic imaging system with respect to the reference area; (iii) determining a first corrective transformation by: capturing, by the first image sensor, a first image comprising an irradiated area; identifying the irradiated area in the first image based on image analysis of the first image; determining the first collimation transformation by mapping the irradiated area onto the reference area; and (iv) determining a second collimation transformation by: capturing, by the second image sensor, a second image comprising an image of an illuminated area on the first image sensor; identifying the illuminated area in the second image based on image analysis of the second image; determining the second collimation transformation by mapping the illuminated area onto the reference area, and wherein the first collimation transformation and the second collimation transformation are used to calculate a corrective transform for changing a system parameter of one or more components of the radiographic imaging system.

In an example, the method further comprises applying the corrective transform to control the system parameter of the one or more components of the radiographic imaging system.

In an example, determining the second collimation transformation comprises transforming the identified illuminated area into a coordinate system utilised by the reference area and the first image.

In an example, arranging the radiographic imaging system in the first configuration comprises manual setting of a collimation parameter for a collimator of the radiographic imaging system based on adjustment of the illuminated area.

In an example, arranging the radiographic imaging system in the first configuration comprises automatic collimation based on the second image sensor data.

In an example, the method further comprises: arranging the radiographic imaging system in a plurality of configurations; repeating steps (ii) to (iv) for the plurality of configurations, to determine a plurality of corresponding first and second collimation transformations for the plurality of configurations; and generating a calibration database comprising the configurations and the corresponding first and second collimation transformations.

In an example, the method further comprises receiving an instruction to change the radiographic imaging system from a current configuration to a new configuration; determining the first and second collimation transformation corresponding to the new configuration based on the calibration database.

In an example, when the new configuration is not included in the calibration database, the determining the first and second collimation transformation comprises: determining a closest configuration to the new configuration comprised in the calibration database; and selecting the collimation transformations corresponding to the closest identified configuration.

In an example, when the new configuration is not included in the calibration database, the determining the collimation transformation comprises: determining a first closest configuration to the new configuration comprised in the calibration database; determining a second closest configuration to the new configuration comprised in the calibration database; interpolating between the collimation transformations corresponding to the first closest configuration and the second closest configuration comprised in the database; and generating the corrective transformation based on the interpolation.

In an example, at least one of the image analysis of the first image and the image analysis of the second image comprises inputting the image to a trained machine learning model.

In an example, the image analysis of the first image comprises recognising an edge of the irradiated area.

In an example, the image analysis of the second image comprises recognising an edge of the illuminated area.

In an example, at least one of the first and second collimation transformations comprises a transformation applied to at least one parameter representing: a offset parameter of a tube head of the radiographic imaging system and the first image sensor; a rotation parameter of the tube head; and collimator opening parameter.

In a related aspect of the invention there is provided a radiographic imaging system for carrying out the above method comprising: a radiation generator configured to irradiate an area of a first image sensor; a light configured to illuminate an area of the first image sensor; a second image sensor for capturing a second image of the first image sensor; and a controller configured to carry out the method of any preceding claim.

It will be appreciated that the invention also extends to radiographic imaging systems corresponding to the method aspects and examples outlined above.

These and other aspects of the present invention will be apparent from and elucidated with reference to the embodiment(s) described hereinafter.

It will be appreciated that the invention also extends to methods corresponding to the apparatus aspects and examples outlined above.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows a schematic of a radiography apparatus;
Fig. 2 shows an example guide pattern, collimation area, and light field; and
Fig. 3 shoes a method for setting corrective transformations.

### DETAILED DESCRIPTION OF EMBODIMENTS

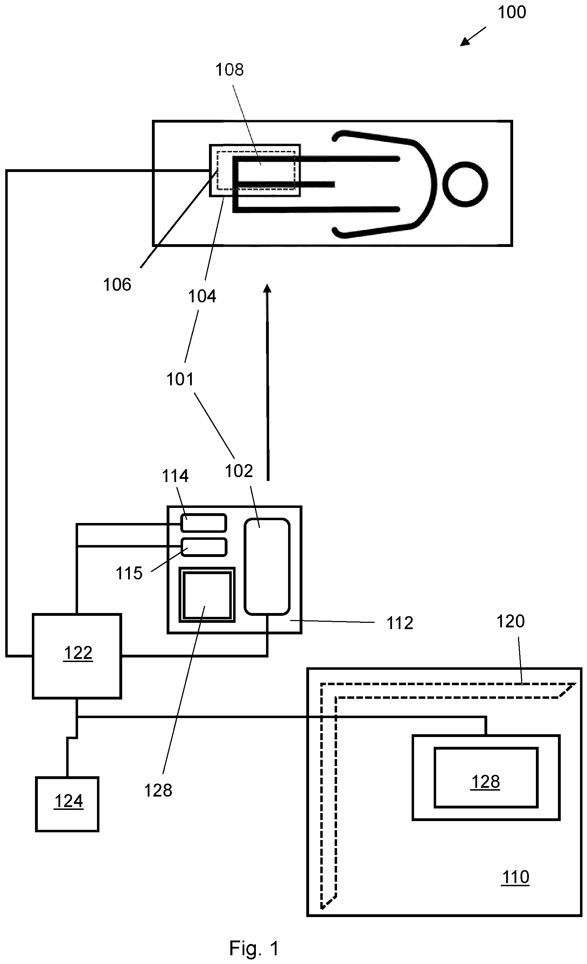

With reference to Fig. 1, there is shown an example radiographic imaging system 100. The radiographic imaging system 100 is based around the emission of ionising radiation and the subsequent imaging of that radiation. For example, the radiographic imaging system 100 may be embodied as an X-ray imaging system, a computed tomography (CT) scan system, a positron emission tomography (PET) scan system, and other variants of imaging systems using ionizing radiation.

The radiographic imaging system 100 is primarily described as a diagnostic system; however, as will be familiar to those in the art, the system 100 could instead be an interventional system.

Suitably, the system 100 comprises a radiographic imaging device 101; for example, comprising an ionising radiation generator/emitter 102 and a radiographic image sensor 104, also termed a first image sensor herein. For example, in the X-ray imaging system, the radiation generator 102 is configured to generate X-rays and the first image sensor 104 is correspondingly configured to capture an X-ray image. If the generator 102 is configured to generate a different form of ionising radiation, the first image sensor 104 is configured accordingly. As will be familiar to those in the art, the arrangement of the radiographic imaging device 102, 104 defines a target area 106 in which a target 108, such as a patient, or part thereof, can be positioned in order to capture a radiographic image based on the penetration of radiation through the target 108 in the target area 106. Put another way, the first image sensor 104 is configured to capture a radiographic image of the target area 106. In some examples, the first image sensor 104 is provided as a separate unit configured to communicate, preferably wirelessly, with other components of the system 100.

In some examples, radiation generator 102 and radiographic image sensor 104 are provided in a fixed arrangement, with the target 108 being moved with respect to the generator 102 and sensor 104 to allow for imaging of different parts of the target 108. Accordingly, in these examples, the target area 106 to be imaged is fixed in position.

In other examples, the radiation generator 102 and radiographic image sensor 104are coupled to suitable movement means such that one or both of the first image sensor 104 and radiation generator 102 may be moved relative to the target 108. That is, in these examples, the target 108 is substantially fixed in position, while the target area 106 to be imaged may be re-positioned according to the moveable arrangement between the first image sensor 104 and radiation generator 102.

In one example of moveable radiographic imaging means 102, 104, the first image sensor 104 is coupled to one or more rails to provide lateral motion, while the generator 102 is coupled to a moveable arm.

In another example of moveable radiographic imaging means 102, 104, the radiation generator 102 is provided on a suitably moveable head part, or tube head, 112. Meanwhile, the first image sensor 104 is provided as a separate unit configured to communicate wirelessly with other components of the system 100. To capture an image of the target 108, the tube head 112 is aligned with the target 108 and the first image sensor 104 freely positioned behind the target 108 to capture an image of the ionising radiation.

In the example shown in Fig. 1, the system 100 comprises a control station 110, from which control parameters and functions of the radiographic imaging device 101 may be set. The control station 110 is disposed separated from the target area 106 of the radiographic imaging device 101 so as to limit the potential for exposure of the operator of the system 100 to radiation. In some examples, the control station 110 is shielded from the imaging device 101 (more specifically, the ionising radiation generator 102 and target area 106 therefor) by a radiation shield 120.

In some examples, the system 100 comprises a display 128 for viewing images captured by the radiographic imaging device 101. As shown in Fig. 1, in some examples, the display 128 may be provided at the control station 110, or on the tube head 112.

The system 100 comprises a second image sensor 114 having a field of view which encompasses the target area 106. That is, the second image sensor 114 can capture images of the target 108 within the target area 106. The operator can use the second image sensor 114 to instruct adjustment of the target 108 and/or system 100 in order to allow for correct imaging of the target 108.

The second image sensor 114 may be configured to capture light at any suitable wavelength that allows for positioning the target 108. In one example, the second image sensor 114 is configured to capture visible light; suitably, in such examples, the second image sensor 114 may be embodied as an RGB camera. In another example, the second image sensor 114 is configured to capture infra-red light, and may therefore be embodied as an infra-red camera. In yet another example, the second image sensor 114 is configured to capture depth information; suitably, in such examples the second image sensor 114 may be part of an RGB depth (RGB-D) camera.

In some examples, the second image sensor 114 is integrated as part of the radiographic imaging device 101. That is, as a component of the respective part of the imaging device 101, such as the X-ray generator 102, or a related component thereof. In the present example, the second image sensor 114 is integrated as part of the tube head 112 of the system 100, such that its position substantially corresponds to the position of the radiation generator 102, and its field of view substantially corresponds with the direction of radiation emission from the generator 102, allowing the second image sensor 114 to capture an image of the target area 106.

In some examples, the system 100 comprises a light 115. The light 115 is configured to emit a visible light beam to indicate a position and orientation of the target area. When the system 100 is in a configuration for capturing an image, the light beam is emitted towards the first image sensor 104, such that the first image sensor 104 has an area illuminated by the light 115. In the present example, the light 115 is integrated as part of the tube head 112 of the system 100, such that its position substantially corresponds to the position of the radiation generator 102 and the second image sensor 114, and the light beam substantially corresponds with the direction of radiation emission from the generator 102, and the field of view of the second image sensor 114. In this way, the illuminated area, can be used to ensure that the system 100 is in a suitable configuration, e.g., to collect an image. In some examples, the light is included in the collimator, and the light beam and radiation beam are emitted from the same or substantially corresponding apertures, so that the light beam corresponds to the radiation beam.

The system 100 comprises a controller 122 (e.g., a processor) configured to control an operating state of the system 100. One example operating state is an image capturing state, i.e., a data acquisition state, in which the device is able to capture an image. That is, the controller 122 is configured to control a data acquisition comprising controlling the radiographic imaging device to capture a radiographic image of the target area 106. Put another way, the controller 122 controls the radiation generator 102 to emit radiation and the first image sensor 104 to record an image of an irradiated area of the first image sensor 104. The controller 122 may also control the second image sensor 114 to also capture an image including the target area 106. The captured second image sensor 114 data may be an image, a video, or the like. Furthermore, at substantially the same time, the controller 122 may control the light 115 to emit the light beam, such that an area illuminated by the light beam is visible in the captured second image sensor data.

Suitably, the data acquisition may be stored in a memory 124. In some examples, the memory 124 is local to the system 100, e.g., an internal memory storage. In other examples, the memory 124 is external to the system 100. In the case of an external memory, the system 100 may comprise suitable transceiver circuitry for wireless communication with the external memory 124.

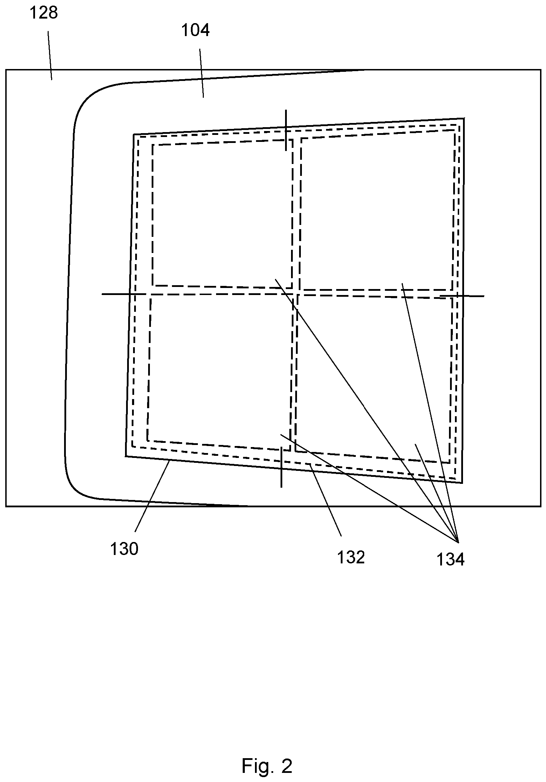

With reference to Fig. 2, there is shown an example of the display 128 during data acquisition for calibration of the system 100. The display 128 shows the captured second image sensor 114 data when the second image sensor is configured to capture an image of the first image sensor 104. Suitably, the display 128 is also configured to display a collimation area 132 and a light field 134 which is visible in the second image sensor data. In some examples, the first image sensor 104 comprises a guide pattern 130 for assisting alignment of the components of the system 100.

It will be appreciated however that the data acquisition may not result in a suitable radiographic image of the target 108. As shown in Fig. 2, in some configurations, the guide pattern 130, the collimation area 132 and the light field 134 may not be aligned. In particular, the guide area 130 defines a maximum collimation area, and in general the collimated area indicated by the light field 134 and the superimposed collimation area 132 (from the system configuration) is smaller than the guide pattern 130. This can lead to problems with the quality of the radiographic image of the target 108, for example, if the wrong part of the target 108 is imaged or if there is excessive radiation to the patient. The radiographic imaging system configuration represents the relative positions and orientations of the components of the system, e.g., the tube head 112, the first image sensor 104 and the collimator shutters.

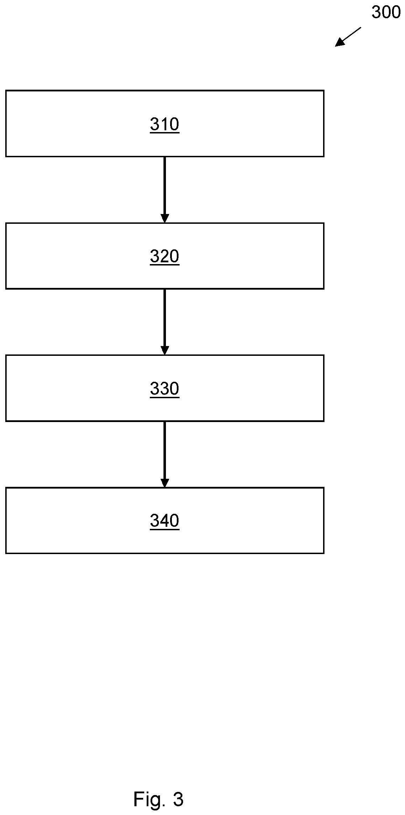

With reference to Fig. 3, to improve the accuracy and consistency of the data acquisition, a computer implemented method 300 for setting corrective transformations for the system 100 is shown. Corrective transformations for changing a system parameter of one or more components of the system 100 are determined and applied to the system parameter of the component(s).

Step 310 comprises arranging the system 100 in a first configuration for capturing an image. In this first configuration, the system 100 has a known geometry, for example, as measured or configured during system installation or as measured or configured once the system 100 is arranged into the first configuration. This known geometry is represented by geometry parameters, which define the position(s) and orientation(s) of the system components with respect to each other, and/or with respect to a system environment. For example, the system geometry parameters may include a distance between the tube head 112 and the first image sensor 104, an angle between the tube head 112 and the first image sensor 104, the relative positions of the camera 114 and the radiation generator 102 with respect to each other and the tube head 112, the position and orientation of the collimator shutters, and the like. In this first configuration, the first image sensor 104 is said to have a first image sensor reference frame, i.e., a first image sensor coordinate system.

To arrange the system 100 into the first configuration, the target area 106 may be set by adjusting a system parameter associated with the collimation. In some examples, this is done by using the light field 134 for guidance. In other examples, this is done by using guidance from the second image sensor 114. For example, an image shown on the display 128 may be used to set relevant collimation parameters, etc, for irradiation of the target area 106.

In step 320, a reference area is obtained using the known geometry of the first configuration. The reference area is the expected target area, i.e., the area that is expected to be irradiated when the first image sensor 104 captures an image. The reference area can be represented by coordinates in the first image sensor coordinate system. In other words, the reference area is determined using the system geometry parameters, i.e., it represents the nominal or theoretical target area based on the system geometry parameters. With reference to Fig. 2, the reference area is shown on the display 128 as the (expected) collimation area 132.

Step 330 comprises determining a first collimation transform, or mapping. In general, the term collimation transform is used for the geometric transform of representations of the collimation area within or between parameter spaces. Suitably, in a perfect system, all representation of the collimation area are equal and the collimation transforms are identity transforms. For example, to determine a first mapping, a first image is captured by the first image sensor 104; an irradiated area is identified in the first image based on image analysis of the first image; and the first collimation transformation is determined by mapping the irradiated area onto the reference area.

The first image is captured by the first image sensor 104 in the data acquisition, as described hereinbefore, in the first configuration. The first image is an image of the received radiation when the radiation does not travel through a target 108 to reach the target area 106. In effect, there is no target 108 in the first image. The first image comprises the irradiated area, i.e., the target area 106.

The irradiated area in the first image is identified based on image analysis of the first image. In some examples, the image analysis of the first image, i.e., the first image analysis, comprises recognising an edge of the irradiated area. The first image analysis can be performed using standard image processing methods, such as edge detection, machine learning techniques, and the like. For example, image analysis of the first image may comprise inputting the first image to a trained machine learning model. In some examples, the irradiated area may be identified from one recognised edge, or more than one edge of the irradiated area may be recognised and the irradiated area can be identified therefrom. In some examples, corners or edges of the irradiated area can be identified, and the corresponding coordinates of the corners or the line of the edge can be identified. The irradiated area is in the first image sensor reference frame, and the corners or edge lines are in the first image sensor coordinate system.

Suitably, the first collimation transformation is defined as the mapping the irradiated area onto the reference area. The mapping may include an affine mapping and/or a projective mapping. In the affine mapping, the irradiated area is mapped onto the reference area using affine transformations such as a translation, a reflection, a scaling, a rotation, and a shearing. In the projective mapping, the irradiated area is mapped onto the reference area using at least one projective transformation. In some examples, based on the mapping, a transformation required to transform the irradiated area onto the reference area can be produced. In some examples, this transformation may be an overall transformation matrix representing the overall transformation required to transform the irradiated area onto the reference area. In other examples, this transformation may be a set of transformation matrices required to transform the irradiated area onto the reference area, and, suitably, an order in which to apply the transformation matrices.

Step 340 comprises determining a second collimation transform. To determine the second collimation transform, a second image is captured by the second image sensor 114; an area illuminated by the light 115 is identified in the second image based on image analysis of the second image; and the second collimation transformation is determined by mapping the illuminated area onto the reference area.

The second image is captured by the second image sensor 114 in the data acquisition, as described herein, in the first configuration. The second image comprises an image of the light field 134 as it illuminates the first image sensor 104.

The illuminated area in the second image is identified based on image analysis of the second image. In some examples, the image analysis of the second image, i.e., the second image analysis, comprises recognising an edge of the illuminated area. The second image analysis can be performed using standard image processing methods, such as edge detection, machine learning techniques, and the like. For example, image analysis of the second image may comprise inputting the second image to a trained machine learning model. In some examples, the illuminated area may be identified from one recognised edge, or more than one edge of the illuminated area may be recognised and the illuminated area can be identified therefrom. In some examples, corners or edges of the illuminated area can be identified, and the corresponding coordinates of the corners or the line of the edge can be identified.

In some examples, the illuminated area is in a second image sensor reference frame, and the corners or edge lines are in a second image sensor coordinate system. The second image sensor reference frame and second image sensor coordinate system are different to the first image sensor reference frame and first image sensor coordinate system.

The second collimation transformation is defined as the mapping the illuminated area onto the reference area. Suitably, in examples in which the reference area is provided in a different coordinate system to that used by the second image sensor, then the mapping of the illuminated area onto the reference area may comprise transforming the illuminated area in the second image sensor coordinate system into the other coordinate system; for example, the first image sensor coordinate system. For example, the coordinates of the corners or edge lines may be transformed from the second image sensor coordinate system into the first image sensor coordinate system. In effect, this enables that the illuminated area and the reference area to be analysed in the same reference frame/coordinate system, thus simplifying the analysis.

The second collimation transformation may include an affine mapping and/or a projective mapping. In the affine mapping, the illuminated area is mapped onto the reference area using affine transformations such as a translation, a reflection, a scaling, a rotation, and a shearing. In the projective mapping, the illuminated area is mapped onto the reference area using at least one projective transformation. In some examples, based on the mapping, a transformation required to transform the illuminated area onto the reference area can be produced. In some examples, this transformation may be an overall transformation matrix representing the overall transformation required to transform the illuminated area onto the reference area. In other examples, this transformation may be a set of transformation matrices required to transform the illuminated area onto the reference area, and, suitably, an order in which to apply the transformation matrices.

With collimation mapping/transforms established, method comprises determining a corrective transform for changing a system parameter of one or more components of the radiographic imaging system. The corrective transform is based on the collimation transforms.

The choice of the corrective transform depends on the way the system is used. The aim of the corrective transform is to ensure that the irradiated area matches the area that has been targeted for imaging. Depending on the use case, the corrective transform is given by a single collimation transform, or a concatenation of collimation transforms.

In one example, the collimated area is defined based on the captured second image sensor 114 data. For example, the operator defines the desired collimation within the camera image ("on-screen-collimation"). As another example, the collimated area may be based on an automatic collimation performed by a processor in which, e.g., the system geometry parameters may be input into a trained machine learning model or other data processing module to output a recommended system parameter. In both cases, the irradiated area in the first image sensor should match the collimated area as shown in the captured second image sensor 114 data. Suitably, the selected collimated area in the captured second image sensor 114 data (either defined by the user or an algorithm) can be transformed into the coordinate system of the first image sensor, i.e. into the reference area using geometric transformations as a result of a camera calibration. Therefore, the corrective transform has to ensure that reference area equals the irradiated area, and consequently the corrective transform equals the first collimation transform (irradiated area to reference area).

In another example, the collimated area is defined by the operator using the light field 134. Thus, the irradiated area needs to match the area illuminated by the light field.

In this example, the corrective transform consists on the combination of two transforms. First, the first collimation transform is applied (irradiated area to reference area) to account for a mismatch between irradiated area and reference area, and afterwards the inverse second collimation transform is applied (reference area to illuminated area) to compensate for a difference between reference area and illuminated area. This corrective transforms can may be applied shortly before the radiation beam is emitted to ensure that the collimation corresponds to the position and area of the light field 134. This automatic shutter adjustment may be triggered by a preparatory signal.

In some examples, a calibration database can be generated. To generate the calibration database, the computer implemented method 300 for setting corrective transformations for the system 100, as shown in Fig. 3 and described hereinbefore, is repeated for various configurations.

To repeat the method 300 to generate a calibration database, steps 310 to 340 are repeated in a plurality of configurations to determine a plurality of corresponding first and second corrective transformations for the plurality of configurations. Ideally, ensure efficient generation of the calibration database, each of the plurality of configurations has at least one system geometry parameter that is different to the corresponding system geometry parameter of the other configurations.

The repeated steps are substantially the same as those described above for the first configuration. For example, in a first repetition of step 310, the system 100 is arranged into a second configuration for capturing an image. In the second configuration, at least one system geometry parameter is different to the system geometry parameter in the first configuration. In the first repetition of step 320, in the reference frame of the first image sensor 104, a reference area is obtained using the known geometry of the second configuration. In the first repetition of step 330, a first collimation transform for the second configuration is determined. In the first repetition of step 340, a second collimation transform for the second configuration is determined. In this way, a first and a second collimation transformation can be generated for the second configuration.

Thus, a calibration database is generated comprising the plurality of configurations and the plurality of corresponding first and second collimation transformations for the plurality of configurations.

The generated calibration database can be used by the system 100 to correct a system parameter. For example, if the system receives an instruction to change from a current configuration to a new configuration, the first and second collimation transformation corresponding to the new configuration can be determined based on the calibration database. A sutable corrective transformations based on the first and second collimation transform can be applied to control a system parameter.

In an example, if the new configuration is not included in the calibration database, the first and second collimation transformations can be determined by determining a closest configuration to the new configuration comprised in the calibration database, and selecting a corresponding closest collimation transformation(s) to calculate the corrective transformation.

The closest configuration to the new configuration comprised in the calibration database is the configuration that is the most similar to the new configuration, in terms of the system geometry parameters. For example, the closest configuration may be the configuration with the most system geometry parameters in common with the new configuration, or the closeness may be based on the similarity of weighted system geometry parameters, such that an increased similarity in certain system geometry parameters may be more significant in determining the closest configuration than that of other system geometry parameters.

In another example, if the new configuration is not included in the calibration database, the first and second collimation transformations can be determined by determining a first closest configuration to the new configuration comprised in the calibration database, determining a second closest configuration to the new configuration comprised in the calibration database, interpolating between the collimation transformations corresponding to the first closest configuration and the second closest configuration comprised in the database, and generating the collimation transformation based on the interpolation.

The first closest configuration to the new configuration comprised in the calibration database is the configuration that is the most similar to the new configuration, in terms of the system geometry parameters. The second closest configuration to the new configuration comprised in the calibration database is the configuration that is the next most similar to the new configuration, in terms of the system geometry parameters. The closeness of the configurations may be determined by the number of system geometry parameters in common with the new configuration, or the similarity of weighted system geometry parameters, as described hereinbefore.

In some examples, the interpolation is a linear interpolation; however, any suitable interpolation can be used.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A computer implemented method (300) for setting a radiographic imaging system (100), wherein the radiographic imaging system comprises a first image sensor (104) and a second image sensor (114), the method comprising:
(i) arranging (310) the radiographic imaging system in a first configuration;
(ii) obtaining (320) a reference area corresponding to the first image sensor (104) and second image sensor (114), based on a known geometry of the radiographic imaging system with respect to the reference area;
(iii) determining (330) a first collimation transformation by:
capturing, by the first image sensor, a first image comprising an irradiated area;
identifying the irradiated area in the first image based on image analysis of the first image;
determining the first collimation transformation by mapping the irradiated area onto the reference area; and
(iv) determining (340) a second collimation transformation by:
capturing, by the second image sensor, a second image comprising an image of an illuminated area (134) on the first image sensor;
identifying the illuminated area in the second image based on image analysis of the second image;
determining the second collimation transformation by mapping the illuminated area onto the reference area, and
wherein the first collimation transformation and/or the second collimation transformation are used to calculate a corrective transform for changing a system parameter of one or more components of the radiographic imaging system.

2. The method of claim 1, further comprising applying the corrective transform to control the system parameter of the one or more components of the radiographic imaging system.

3. The method of claim 1 or 2, wherein determining the second collimation transformation comprises transforming the identified illuminated area into a coordinate system of the obtained reference area.

4. The method of any of claims 1 to 3, wherein arranging the radiographic imaging system in the first configuration comprises manual setting of a collimation parameter for a collimator of the radiographic imaging system.

5. The method of any of claims 1 to 3, wherein arranging the radiographic imaging system in the first configuration comprises automatic collimation based on adjustment of the illuminated area.

6. The method of any preceding claim, further comprising:
arranging the radiographic imaging system in a plurality of configurations;
repeating steps (ii) to (iv) for the plurality of configurations, to determine a plurality of corresponding first and second collimation transformations for the plurality of configurations; and
generating a calibration database comprising the configurations and the corresponding first and second collimation transformations.

7. The method of claim 6, further comprising:
receiving an instruction to change the radiographic imaging system from a current configuration to a new configuration;
determining the first and second collimation transformation corresponding to the new configuration based on the calibration database.

8. The method of claim 7, wherein, when the new configuration is not included in the calibration database, the determining the first and second collimation transformation comprises:
determining a closest configuration to the new configuration comprised in the calibration database; and
selecting a corresponding closest collimation transformations to calculate the corrective transformation.

9. The method of claim 7, wherein, when the new configuration is not included in the calibration database, the determining the collimation transformation comprises:
determining a first closest configuration to the new configuration comprised in the calibration database;
determining a second closest configuration to the new configuration comprised in the calibration database;
interpolating between the collimation transformations corresponding to the first closest configuration and the second closest configuration comprised in the database; and
generating the corrective transformation based on the interpolation.

10. The method of any preceding claim, wherein at least one of the image analysis of the first image and the image analysis of the second image comprises inputting the image to a trained machine learning model.

11. The method of any preceding claim, wherein the image analysis of the first image comprises recognising an edge of the irradiated area.

12. The method of any preceding claim, wherein the image analysis of the second image comprises recognising an edge of the illuminated area.

13. The method of any preceding claim, wherein the corrective transformation comprises a transformation applied to at least one parameter representing:
a offset parameter of a tube head (112) of the radiographic imaging system and the first image sensor;
a rotation parameter of the tube head; and
a collimator opening parameter.

14. A radiographic imaging system (100) for carrying out the method (300) of any preceding claim comprising:
a radiation generator (102) configured to irradiate an area of a first image sensor (104);
a light (115) configured to illuminate an area of the first image sensor;
a second image (114) sensor for capturing a second image of the first image sensor; and
a controller (122) configured to carry out the method of any preceding claim.
